(19)

Europäisches
Patentamt
European
Patent Office
Office européen
des brevets

(11) **EP 2 444 064 A1**

(12) **EUROPEAN PATENT APPLICATION**

(43) Date of publication:
**25.04.2012 Bulletin 2012/17**

(51) Int Cl.:
***A61K 9/00*** *(2006.01)*

(21) Application number: **10290570.0**

(22) Date of filing: **22.10.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Meliatys**
**13790 Rousset (FR)**

(72) Inventor: **Kirkorian, Joël Sylvain Michel**
**84400 Apt (FR)**

(74) Representative: **Hirsch & Associés**
**58, avenue Marceau**
**75008 Paris (FR)**

(54) **Process for making multiparticulate gastroretentive dosage forms**

(57)    The instant invention relates to a process for making inherent low density particles, comprising the steps of (i) providing a powder mixture comprising a swelling agent; (ii) granulating the powder of step (i) with a granulating solution comprising a lipophilic agent into granules and (iii) drying the granules of step (ii). The instant invention further relates to multiparticulate oral gastro-retentive dosage forms comprising the inherent low density particles obtainable by the process.

FIGURE 1

EP 2 444 064 A1

**Description**

FIELD OF THE INVENTION

The present invention relates to novel pharmaceutical compositions which are retained in the stomach or upper gastrointestinal tract for a controlled delivery of a drug. The present invention also provides methods of preparation as well as methods of using these dosage forms in therapeutic treatments.

BACKGROUND OF THE INVENTION

Therapeutic agents see their efficiency intimately related to their method of administration. When taken orally, a drug interacts with specific absorption sites located in different portions throughout the gastrointestinal tract (GI), resulting in that certain agents are only absorbed in the stomach, the upper or lower intestine. Therefore, because the drugs are not absorbed uniformly all over the length of the GI tract, the rate of absorption may not be constant and does not allow a most efficient treatment. These may significantly be improved when the method of administration provides a controlled delivery of the active ingredient towards the only implicated sites.

For example, it may be significant to prolong the residence time specifically in the stomach in the case of drugs which are only locally active such as anti-acids, have an absorption window in the stomach or in the upper intestine such as L-Dopa or riboflavin, are unstable in the intestinal or colonic environment such as captopril or exhibit low solubility at high pH values such as diazepam, or verapamil. This may be also important in treatments of microorganisms, which colonize the stomach since the three main factors reducing luminal delivery of drugs to them are gastric emptying, gastric acidity and the epithelial mucus layer. These forms may also be used to release a biomarker to monitor and identify gastric conditions.

While the existing immediate release forms provide the disadvantage of repeated administration of a medicament as well as fluctuations in drug plasma levels, controlled drug delivery systems were significantly developed.

They allow the delivery of a therapeutic agent in such way that the level of the drug is maintained within a particular window as long as the form continues to deliver the drug at a constant rate. Also, apart from reducing the required frequency of administration or maintaining safe blood levels, there are other benefits associated with the intake of controlled release forms such as the reduction of the severity of side effects.

A large variety of controlled release forms have already been disclosed, as summarized in "Gastroretentive drug delivery systems", by Alexander Streubel, Juergen Siepmann & Roland Bodmeier, Expert Opin. Drug Deliv. (2006) 3(2):217-233, or in "Gastroretentive dosage forms: Overview and special case of Helicobacter Pylori", J. Control. Rel., 111 (2006) 1-18 by Bardonnet et al. They are based on different modes of operation and accordingly have been variously named, for example, as dissolution controlled systems, diffusion controlled systems, ion exchange resins, osmotically controlled systems, erodible matrix systems, pH independent formulations, bioadhesive forms, low density systems, swelling forms and the like.

The low density systems particularly, float once in contact with the gastric juice and allow prolonged residence time into the stomach by preventing premature emptying through the pylorus. They are usually made of biodegradable materials which disintegrate after a determined period of time and the residual form is then emptied from the stomach. Floating properties of drug delivery systems can be based on several principles, including inherent low density, low density due to swelling or to gas generation.

The swelling systems for example, not only see their size increase above the diameter of the pylorus which results from the unfolding of complex geometric shapes, or the expansion of swellable excipients, but also see their density decrease to provide floating properties. For the gas generating systems, the low density is obtained from the formation of carbon dioxide within the device following contact with body fluids. Some of these dosage forms already exist and usually associate both swelling and gas generation phenomena. Some of them are currently being tested clinically such as Cipro XR®, Xatral® OD, or have already received the approval of a Drug Regulatory Administration such as Glumetza® or Proquin XR®. They however have the draw back not to swell/float directly following the administration, as it takes time for the systems to reach the desired size, and even longer when it is an effervescent form because of the gas generation process.

More advantageously, in inherent low density systems, the floating properties are effective as soon as the form is swallowed, allowing for substantially no lag time. They are generally provided by entrapment of air, incorporation of low density materials, with foam powders, or combinations thereof.

For example, Desai and Bolton in US 4,814,179 developed a moulded agar gel tablet with oil and air, which replaced evaporated water following drying. The process for manufacturing involves the steps of forming an emulsion, from an oily composition of the active and an aqueous solution of agar gel. The emulsion is poured into a mould and subsequently dried.

Krögel and Bodmeier proposed in "Development of a multifunctional matrix drug delivery system surrounded by an impermeable cylinder", J. Control. Release (1999) 61:43-50, a floating device consisting of an impermeable hollow polypropylene cyclinder, containing two drug matrix tablets, each of them closing one end of the cylinder, so that an air-filled space was created in between, resulting in a low density system.

More recently, developments led to single unit and multiparticulate systems containing highly porous polypropylene foam powder and matrix forming polymers, which are said to provide a low density, excellent *in vitro* floating behaviour and broad spectrum of release patterns. See for example WO 89/06956, disclosing a floating drug wherein a porous structural element, such as a foam or a hollow body is placed within a matrix, and optionally compressed into a tablet dosage form. See also Streubel, Siepmann & Bodmeier, "Floating matrix tablets based on low density foam powder", Eur. J. Pharm. Sci. (2003) 18:37-45, or Int. J. Pharm. (2002) 241:279-292, which provides examples of such matrix forming polymers: hydroxypropyl methylcellulose, polyacrylates, sodium alginates, corn starch, carrageenan, gum guar, gum arabic, Eudragit®RS, ethyl cellulose, or poly methyl methacrylate.

Another multiple unit gastroretentive drug delivery system containing air compartments was disclosed by Iannucelli et al., wherein each single unit consisted of a calcium alginate core, separated by an air compartment formed during a drying step, from a calcium alginate or calcium alginate/polyvinyl alcohol membrane. It is said to show both good *in vitro* and *in vivo* buoyancy behaviour and suitable drug release patterns were observed when both the core and the membranes were loaded with a solid dispersion of drug/polyvinylpyrrolidone.

Finally, some other bead formulations containing air compartment were developed by incorporation of air bubble and air filled hollow spaces within the system. These are disclosed by Bulgarelli et al., in "Effect of matrix composition and process conditions on casein-gelatin beads floating properties", Int. J. Pharm. (2000) 198:157-165, and by Talukder et Fassihi, "Gastroretentive delivery systems: hollow beads", Drug Dev. Ind. Pharm. (2004) 30:405-412. Floating properties however depend on the filling state of the stomach.

Most of the above compositions incorporate air into the dosage form via a specific vehicle, e.g. a prefabricated foam product (e.g. polypropylene foam).

Still, the above technical solutions are not applicable to any type of active ingredients, do not accommodate any loading rate, and are difficult to carry out.

Thus, there is still a need for another inherent sustained release form which provides improved properties and bioavailability.

In the European patent application EP 2 133 071, which is incorporated by reference into the present application, a new process allowed preparing monolithic gastro-retentive forms that were provided with an inherent low density. Different types of active ingredients could be overgranulated into a paste with hydrophobic excipients. Upon drying, the cavities that formed within the paste led to a final material that was able to float upon contact with the gastric juice. Thus, the air incorporated into the dosage form came from the water of the manufacturing process itself. However, this process necessitates constraining conditions of manufacture and the overgranulated paste remains difficult to mould into appropriate forms before the drying step. In fact, the amount of water that is necessary to obtain the appropriate low density could not be incorporated without reaching the overgranulated paste. Thus, the process of the prior art suffers from drawbacks when being carried out.

Particularly, there is a need to provide an improved process to manufacture a form that floats immediately once in contact with the gastric juice in order to avoid any premature emptying through the pylorus. This process should also be compatible with different active ingredients, at different concentrations and provide forms that have a good bioavailability and optimize the therapeutic efficiency of the drug.

Finally, considering the complexity of the technology of existing forms, there is still a need for systems that can be easily manufactured at an industrial scale.

SUMMARY OF THE INVENTION

One aspect of the invention is directed to a process for making low density particles, comprising the steps of:

    (i) providing a powder mixture comprising a swelling agent;
    (ii) granulating the powder of step (i) with a granulating solution comprising a lipophilic agent into granules;
    (iii) drying the granules of step (ii).

According to another embodiment, the process further comprises the step (iv) of compressing the granules of step (iii).

According to another embodiment, the process f claims 1 to 2, further comprises the step (v) of coating the granules resulting from step (ii) or step (iii).

According to another embodiment, the active ingredient is added into the starting powder of step (i) and/or the granulating solution of step (ii), preferably into the starting powder of step (i) and/or is laid on the granules obtained in step (iii).

According to another embodiment, a binder is added with the starting material in step (i) and/or the granulating solution of step (ii), preferably into the starting powder of step (i).

According to another embodiment, the swelling agent is a cellulose derivative having a molecular weight from 4,000 to 2,000,000, hydroxymethylcellulose, hydroxyethylcellulose hydroxypropyl methylcellulose, superporous hydrogels; polyethylene oxides, polyethylenes; polypropylenes; polyvinyl chlorides; polycarbonates; polystyrenes; polyacrylates; carboxyvinyl polymers; polyvinyl alcohols; glucans; scleroglucans; chitosans; mannas; galactomannans; gums; xantan gums; carrageenans; amylase; alginic acids, acrylates, methacrylates, acrylic/methacrylic copolymers, polyanhydrides, polyamino acids, methyl vinyl ethers/maleic anhydride copolymers, carboxymethylcellulose, carboxymethylcellulose derivatives and copolymers thereof or a water soluble resin and mixture thereof, most preferably selected among polyethylene oxides having a molecular weight of at least 1,000,000 and hydroxypropyl methylcellulose with a molecular weight of at least 100,000 and combinations thereof.

According to another embodiment, the granulating solution is an aqueous solution or dispersion, an organic solvent, a hydrophobic liquid or water, preferably water.

According to another embodiment, the lipophilic agent comprises one or more highly lipophilic excipients selected from the group consisting of hydrophobic dusty powders and lipidic excipients, preferably from the group consisting of talc, hydrophobic silica, magnesium stearate, glicerides, fatty acid esters or fatty acid, preferably talc, stearic acid glicerides and mixtures thereof.

According to another embodiment, the particles comprise:

- from 0.01 to 90%, preferably 20 to 90% of active ingredient;
- from 1 to 99%, preferably of swelling agent;
- from 1 to 60%, preferably 5 to 50% of lipophilic agent; and optionally;
- from 1 to 20%, preferably 2 to 15% of binder.

According to another embodiment, the active ingredient is selected from the group consisting of AIDS adjunct agents, alcohol abuse preparations, Alzheimer's disease management agents, amyotrophic lateral sclerosis therapeutic agents, analgesics, anesthetics, antacids, antiarythmics, antibiotics, anticonvulsants, antidepressants, antidiabetic agents, antiemetics, antidotes, antifibrosis therapeutic agents, antifungals, antihistamines, antihypertensives, antiinfective agents, antimicrobials, antineoplastics, antpsychotics, antiparkinsonian agents, antirheumatic agents, appetite stimulants, appetite suppressants, biological response modifiers, biologicals, blood modifiers, bone metabolism regulators, cardioprotective agents, cardiovascular agents, central nervous system stimulants, cholinesterase inhibitors, contraceptives, cystic fibrosis management agents, deodorants, diagnostics, dietary supplements, diuretics, dopamine receptor agonists, endometriosis management agents, enzymes, erectile dysfunction therapeutics, fatty acids, gastrointestinal agents, Gaucher's disease management agents, gout preparations, homeopathic remedy, hormones, hypercalcemia management agents, hypnotics, hypocalcemia management agents, immunomodulators, immunosuppressives, ion exchange resins, levocarnitine deficiency management agents, mast cell stabilizers, migraine preparations, motion sickness products, multiple sclerosis management agents, muscle relaxants, narcotic detoxification agents, narcotics, nucleoside analogs, non-steroidal anti-inflammatory drugs, obesity management agents, osteoporosis preparations, oxytocics,parasympatholytics, parasympathomimetics, phosphate binders, porphyria agents, psychotherapeutic agents, radio-opaque agents, psychotropics, sclerosing agents, sedatives, sickle cell anemia management agents, smoking cessation aids, steroids, stimulants, sympatholytics, sympathomimetics, Tourette's syndrome agents, tremor preparations, urinary tract agents, vaginal preparations, vasodilators, vertigo agents, weight loss agents, Wilson's disease management agents, and mixtures thereof, and preferably is selected from the group consisting of abacavir sulfate, abacavirsulfate / lamivudine / zidovudine, acetazolamide, acetaminophen, acyclovir, albendazole, albuterol, aldactone, allopurinol BP, Aluminium carbonate, Aluminium hydroxide, amoxicillin, amoxicillin / clavulanate potassium, amprenavir, artesunate, atovaquone, atovaquone and proguanil hydrochloride, atracurium besylate, baclofen, barium sulfate, beclomethasone dipropionate, berlactone betamethasone valerat, betaine, Bismuth subsalicylate, bupropion hydrochloride, bupropion hydrochloride SR, Calcium carbonate, carbamazepin, carbidopa, carvedilol, caspofungin acetate, cefaclor, cefazolin, ceftazidime, céfuroxime, chlorambucil, chloroquin, chlorpromazine, cimetidine, cimetidine hydrochloride, ciprofloxacine, cisatracurium besilate, clobetasol propionate, co-trimoxazole, colfoscerilpalpitate, dextroamphetamie sulfate, dioxin, dihydroxyartemisinin, doxycycline, enalapril maleat, epoprostenol, esomepraxole magnesium, fluticasone propionate, furosemide, gabapentin, glitazones, hydrotalcite, hydrocodone hydrochlorothiazide/triamterene, lamivudine, lamotrigine, levodopa, lithium carbonate, lomefloxacine, losartan potassium, Magnesium aluminate monohydrate melphalan, mercaptopurine, mefloquine mesalazine, metformine, morphin, mupirocin calcium cream, nabumetone, naratriptan, norfloxacine, ofloxacine, omeprazole, ondansetron hydrochloride, ovine, oxiconazole nitrate, oxycodone, paroxetine hydrochloride, pefloxacine, piroxicam, prazodin, prochlorperazine, procyclidine hydrochloride, pyrimethamine, ranitidine bismuth citrate, ranitidine hydrochloride, repaglinide, rofecoxib, ropinirole hydrochloride, rosiglitazone maleat, salmeterol xinafoate, salmeterol, fluticasone propionate, Sodium bicarbonate, sterile ticarcillin disodium/clavulanate potassium, simeticon, simvastatin, spironolactone, statins, succinylcholine chloride, sumatriptan, tapentadol, thioguanine, tirofiban hydrochloride, topotecan hydrochloride, tramadol, tranylcypromine sulfate, trifluoperazine hydrochloride, valacyclovir hydrochloride, vinorelbine, zaleplon, zanamivir, zidovudine, zidovudine or lamivudine, corresponding salts thereof, or mixtures thereof, and is most preferably metformin, glitazones, tramadol, tapentadol, oxycodone, hydromorphone and especially with acetaminophen.

According to another embodiment, the inherent low density particles have a density below 1, preferably below 0.9 and more preferably below 0.8, and preferably have an intrinsic porosity.

According to another embodiment, the inherent low density particles are further processed into an oral solid gastro-retentive dosage form in a tablet, a capsule or a sachet.

Another aspect of the invention is directed to a multiparticulate oral gastro-retentive dosage form in a tablet, a capsule or a sachet, comprising low-density particles obtainable by the process according to the invention.

According to another embodiment, the multiparticulate sustained release dosage form is in the form of a tablet.

According to another embodiment, the particles have a porosity of from 10 to 80%, preferably of from 20 to 70% of the volume of the form.

BRIEF DESCRIPTION OF THE DRAWINGS

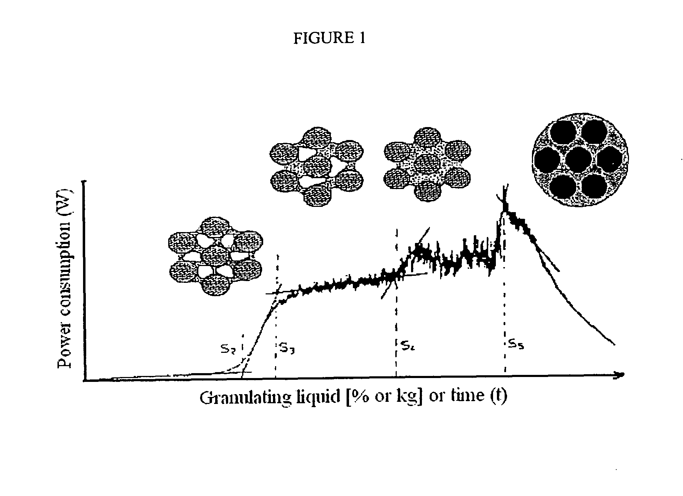

Figure 1 is a diagram representing the different stages of a granulating process according to the amount of granulating liquid which is added to the initial powder. Stage 3 represents granulated particles and stage 4, the overgranulated phase.

DETAILED DESCRIPTION OF EMBODIMENTS OF THE INVENTION

According to a first aspect, the invention is directed to an improved process for the preparation of multiparticulate low density gastro-retentive forms.

Granulating techniques are extensively used in industry and particularly for the preparation of pharmaceutical dosage forms. To perform a wet granulation for example, liquid solvents having low viscosity (usually water) and possibly containing binders are added to the bulk powder in a fluidized bed or a high shear mixer or impeller mixer, so that the solid particles can link to each other and form agglomerates and granules.

The granulation phenomenon is represented in figure 1, showing the formation of bridges between the solid particles as the amount of the granulating solution increases. In the last stage, the saturation is reached once the interparticular void spaces have been filled up (step IV), and finally the overgranulated solid system turns into a liquid paste. Each step represents a progressive increase in the moisture content, agglomeration mechanism is a gradual change from a triphasic stage (air - liquid - solid) in which granules are in pendular (I) and funicular (II) states to a biphasic (liquid - solid) in which the granules are in the capillary (III) and droplets (IV) state.

It is now known that, when using the granulating liquid as a vehicle for incorporating a large amount of a hydrophobic dusty powder, by overgranulating the particles, it is possible to obtain a solid floating dosage form based on low density. The use of such granulating liquid with a dispersion of hydrophobic dusty allows obtaining a high porosity and then obtaining a low intrinsic density. The incorporation of the hydrophobic dusty powder into granulating solution create a new "granulation dispersion" which allows obtaining floating granules.

However, it has surprisingly been found that the large amounts of solution that are required may in fact be incorporated to the granules without having to reach the overgranulated state. It has indeed been discovered that the use of swelling agents in the composition of the granules allow absorbing high amounts of water that are sufficient to create an inherent porosity and low density upon drying. It is therefore surprising to see that it remains possible to remain at the solid state while incorporating the granulating liquid. The overgranulated paste of prior art, corresponding to a liquid external phase now becomes a semi-overgranulated granule corresponding to a solid external phase with a large amount of water and air entrapped in said granules.

Therefore, it is now possible to avoid the constraining conditions of the processes of the prior art, i.e. the requirements of high energy for mixing and inversing the granulating phase in a high shear mixer and difficulties of molding the liquid phase. The invention thus resides in the combined effects of an efficient wetting solution on the one hand and of an excipient which is capable of absorbing several times its weight of the solution in the other hand. While the wetting solution allows reaching the inherent low density, the absorbing excipient allows putting enough wetting solution to the system.

A first step of the process is to provide a powder mixture comprising the swelling agent in a powder form. The powder may further contain a binder. They are admixed in the desired proportions and eventually dry blended to provide a homogeneous powder mixture. In this case, it may be preferred that the rotating speed is adapted to avoid the dispersion of the components onto the walls of the vessel. Preferably, if the active ingredient is intended to be dispersed within the intrinsic low density and highly porous material, it should also be loaded into the powder mixture with optionally the other adjuvants.

On the other hand, a granulating suspension comprising lipophilic agent dispersed therein. This suspension can further contain part of the binders which is solubilized therein.

A second step of the process is the granulation of the previous powder mixture with the previous granulating solution, preferably an aqueous solution, to be performed until granules are formed. Suitable installation may be any conventional setting to the man skilled in the art, such as a planetary mixers or high shear mixer with an impeller. Due to the fact that an overgranulated paste does no longer have to be reached to provide inherent low density particles, mixing conditions do not need to be as constraining as in the prior art.

A particularly suitable granulating liquid is water although any aqueous solution may be used. Any other conventional granulation liquids may be suitable, such as organic solvents, or hydrophobic materials that are liquid at room temperature.

The wetting solution can comprise part or, all of the binder and/or part or all of the active ingredient, if it is water soluble and/or part or all of a surfactant if any. The weight ratio of solution: powder to reach is strongly dependant of the global solubility of the powder mixture and is generally higher than 0.3, and typically comprised in the range of about 0.3:1 to about 3:1, preferably of about 0.7:1 to about 2:1.

According to a preferred method of granulation, the aqueous solution is added drop wise. The mixing is then continued until the mixture turns into granules of an appropriate size. Once again the rotating rate does no longer need to be as high as in the processes of the prior art where the overgranulated paste required rotating rates of from 150 to 1500 rpm. The rotating speed and the wetting liquid addition rate can be adapted one to the other. Typically, the granulation step is achieved when the powder mixture reaches the « snowball aspect » well known by the man skilled in the art. It is important to notice that, regarding the specific composition of the invention and especially regarding the swelling agents, it is possible to continue the incorporation of the wetting suspension without reaching the overgranulated paste. Thus, the granulation step can be adapted to obtain the final properties that are required for the granules, especially for the physicochemical properties and taking into account the API in the granules.

This critical amount of granulating liquid usually corresponds to about 80% by weight of the weight of the starting material. Upon drying, the liquid will leave cavities inside the granules to provide an inherent low density to the particles.

A last step of the process would be finally to extract the granulating solution up to dryness, to a maximum water content of about 3% of the global composition. This can be performed by lyophilisation methods or by any other conventional techniques to the man skilled in the art such as drying in a ventilated oven, in the mixer, in a fluid bed system etc.

In order to improve the porosity of the final resulting material, it may be particularly interesting to add at any stage of the process prior to the drying step a gas forming agent which will incorporate further air into the granules.

Further processing steps may be performed in order to manufacture multiparticulate gastro-retentive dosage forms according to conventional techniques. For example, the resulting granules can be processed into compressed tablets, sachet or capsules.

In another embodiment of the invention, the granules can be compressed in tablets without loosing their intrinsic low density.

In order to facilitate this further step, additional adjuvants can be added to the composition into the initial powder mixture, during the manufacture or at the end, such as protective agents, lubricants, anti-static agents or glidants. Optionally, the dried granules can also be coated before further processing into gastro-retentive dosage forms.

In the framework of this invention, the expression "protective agents" means any excipient which will protect the granules during compression onto a tablet or during filling capsules or sachets. The protection means the ability to absorb the main effects of the compression and then to protect the porosity of the granules. In such cases, the disintegration of the tablets, capsules, sachets will lead, on an appropriate speed, to the release of the floating gastro retentive granules.

In the framework of this invention, the expression "lubricant" means any excipient which ease ejection of the tablet from the tableting dye in which it is formed by compression and improves the flow properties of the composition in powder or granule. Examples of lubricants are Silicon Dioxyde, talc, Sodium stearyl fumarate, magnesium stearate and glyceryl behenate, and mixtures thereof.

According to another alternative, the inherent low density granules obtained according to the present process may be coated onto a core to provide the resulting dosage form with low density properties.

Therefore, the multiparticulate gastro-retentive forms according to the invention can be advantageously prepared by improved techniques which are easily operated at an industrial scale.

According to another embodiment, the API is contained in an outer layer surrounding a core having the advantageous intrinsic properties of the dosage form, or eventually in one or more additional layers.

Thereby, the release of the drug may be sustained, controlled or extended depending on the structure of the dosage form, and the type of ingredients and/or adjuvants that are used.

EP 2 444 064 A1

In another embodiment, one API may be dispersed within the dosage form while another is present in an outer layer. Preferably, the API in the outer layer is in a form that is an immediate release form. One example would be a dosage form with an antibiotic (eg. Ciprofloxacin) in the core and a benzimidazole (eg. Omeprazole) in the outer immediate release layer.

In addition, in accordance with the preferred embodiment where the API is dispersed and processed with the lipophilic excipients, the resulting granulated material provided with high porosity and low density may be used to manufacture dosage form. Indeed, according to these different embodiments, any oral dosage form comprising this material will have the required floating properties.

Unlike the existing sustained release systems which are designed for a specific drug to be administered, the solid dosage form of the invention may advantageously be associated with any suitable active ingredient (API) that provides a therapeutical effect.

Therefore, the invention allows the manufacture of sustained, controlled, extended, release granules according to the amount of the lipophilic agent and the other excipients. But in another embodiment of the invention, it is possible to adapt the dissolution profiles regarding the API properties and their amount in the granules. These adaptations cannot be only achieved by the granulating step but they can take place before or after the granulating step. For example, in the case of high content API which is very soluble in water or gastric juice, it is possible to make a first granulation or coating of the API (with excipients) with a sufficient particle size and then to go to the granulation step with the coated API. Then the release will be achieved in a longer period and the granulated/coated material will have no impact on the final low density of the granules as the granulation step is strongly related to the formulation of the floating material. On the same approach, the granulation step might be the first step and then a further granulating/coating step may be achieved on the dried granules, in order to adapt the release of the API in the granules, tablets, sachets, capsules, etc. These first and final steps may be achieved alone or together with the low density granulation step and then might be applied on different APIs. The invention is ideal for oral delivery of a wide range of molecules characterized by a narrow absorption window and is particularly effective with water soluble and poorly soluble molecules with different physicochemical properties and molecular sizes.

Examples of suitable API without being limitative may be any relating to one or more of the: AIDS adjunct agents, alcohol abuse preparations, Alzheimer's disease management agents, amyotrophic lateral sclerosis therapeutic agents, analgesics, anesthetics, antacids, antiarythmics, antibiotics, anticonvulsants, antidepressants, antidiabetic agents, antiemetics, antidotes, antifibrosis therapeutic agents, antifungals, antihistamines, antihypertensives, antiinfective agents, antimicrobials, antineoplastics, antpsychotics, antiparkinsonian agents, antirheumatic agents, appetite stimulants, appetite suppressants, biological response modifiers, biologicals, blood modifiers, bone metabolism regulators, cardioprotective agents, cardiovascular agents, central nervous system stimulants, cholinesterase inhibitors, contraceptives, cystic fibrosis management agents, deodorants, diagnostics, dietary supplements, diuretics, dopamine receptor agonists, endometriosis management agents, enzymes, erectile dysfunction therapeutics, fatty acids, gastrointestinal agents, Gaucher's disease management agents, gout preparations, homeopathic remedy, hormones, hypercalcemia management agents, hypnotics, hypocalcemia management agents, immunomodulators, immunosuppressives, insomnia, ion exchange resins, levocarnitine deficiency management agents, mast cell stabilizers, migraine preparations, motion sickness products, multiple sclerosis management agents, muscle relaxants, narcotic detoxification agents, narcotics, nucleoside analogs, non-steroidal anti-inflammatory drugs, obesity management agents, osteoporosis preparations, oxytocics,parasympatholytics, parasympathomimetics, phosphate binders, porphyria agents, psychotherapeutic agents, radio-opaque agents, psychotropics, sclerosing agents, sedatives, sickle cell anemia management agents, smoking cessation aids, steroids, stimulants, sympatholytics, sympathomimetics, Tourette's syndrome agents, tremor preparations, urinary tract agents, vaginal preparations, vasodilators, vertigo agents, weight loss agents, Wilson's disease management agents, and mixtures thereof.

Without being limitative, suitable active ingredients may thus be one or more selected from: abacavir sulfate, abacavirsulfate/lamivudine/zidovudine, acetaminophen, acetazolamide, acyclovir, albendazole, albuterol, aldactone, allopurinol BP, amoxicillin, amoxicillin/clavulanate potassium, amprenavir, artesunate, atovaquone, atovaquone and proguanil hydrochloride, atracurium besylate, baclofen, beclomethasone dipropionate, berlactone betamethasone valerat, betaïne, bupropion hydrochloride, bupropion hydrochloride SR, carvedilol, caspofungin acetate, carbamazepin, carbidopa, cefaclor, cefazolin, ceftazidime, céfuroxime, chlorambucil, chlorpromazine, cimetidine, cimetidine hydrochloride, ciprofloxacine, cisatracurium besilate, clobetasol propionate, co-trimoxazole, codeine, colfoscerilpalpitate, dextroamphetamie sulfate, dihydroxyartemisinin, dioxin, doxycycline, enalapril maleat, epoprostenol, esomepraxole magnesium, fluticasone propionate, furosemide, gabapentin, glitazones, hydrochlorothiazide/triamterene, hydrocodone, hydromorphone, lamivudine, lamotrigine, levodopa, lithium carbonate, lomefloxacine, losartan potassium, melphalan, mercaptopurine, mesalazine, metformin, morphin, mupirocin calcium cream, nabumetone, naratriptan, norfloxacine , ofloxacine, omeprazole, ondansetron hydrochloride, ovine, oxiconazole nitrate, oxycodone, paroxetine hydrochloride, pefloxacine, piroxicam, prazodin, prochlorperazine, procyclidine hydrochloride, pyrimethamine, ranitidine bismuth citrate, ranitidine hydrochloride, Repaglinide, rofecoxib, ropinirole hydrochloride, rosiglitazone maleat, salmeterol xinafoate, salmeterol, fluticasone propionate, sterile ticarcillin disodium/clavulanate potassium, simeticon, simvastatin, spironolactone, statins, succinylcholine chloride, sumatriptan, tapentadol, thioguanine, tirofiban hydrochloride, topotecan hydrochloride, tramadol, tranylcypromine sulfate, trifluoperazine hydrochloride, valacyclovir hydrochloride, vinorelbine, zaleplon, zanamivir, zidovudine, zidovudine or lamivudine, corresponding salts thereof, or mixtures thereof.

Preferably, the active ingredient is one or more of the API chosen from the group consisting of the antibacterial agents, or the antibiotics, such as norfloxacine, ofloxacine, ciprofloxacine, pefloxacine, lomefloxacine, quinolones, ceflaclor or pharmaceutically acceptable salts thereof, the analgesics, such as tramadol, morphine or pharmaceutically salts thereof, the anti-acids such as simethicones, and the anti-diabetes, such as metformin or pharmaceutically acceptable salts thereof. These drugs exhibit higher therapeutical effects when absorbed in the upper intestine and stomach.

A most preferred API would be those that provide beneficial therapeutic effects for urinary infections or diseases, such as ciprofloxacine, lomefloxacine, ofloxacine, or pharmaceutically acceptable salts thereof, or diabetes, such as metformine, or any of their pharmaceutically acceptable salts.

Further most preferred API would be those that provide beneficial therapeutic effects for diabete type 2 and especially combo with gastro retentive metformin, metformin XR 500, 750, 850 and 1000 mg, and DPP-4 inhibitors, SGLT-2 and Glitazones.

Further most preferred API would be opioids like tramadol, tapentadol, oxycodone, hydromorphone, codeine and hydrocodone and especially combo with acetaminophen.

The amount of the active ingredient in the pharmaceutical compositions of the present invention will be a therapeutically effective amount. The dosage form of the invention allows high drug loads compared to the existing sustained release forms and a therapeutically effective amount will generally be an amount within the range of from about 0.01 to about 90%, preferably within the range of from about 20 to about 90% and more preferably of from about30 to about 85% by weight of the composition. It is understood that higher or lower weight percentages of the active ingredient may be present in the pharmaceutical compositions. By "therapeutically effective amount" as used herein is meant an amount of active component in the pharmaceutical compositions of the present invention which is effective to beneficially treat the patient in need thereof.

As used therein, "swellable excipients" is intended to mean any excipient which once in contact with the gastric juice increase in size and has the ability to absorb the granulating liquid but also to release the liquid when processed in a drying step. The swelling agent is responsible for absorbing the amounts of liquid which creates the cavities within the structure of the particles once dried.

Appropriate swelling agent may be selected from the group consisting of cellulose derivatives having a molecular weight from 4,000 to about 2,000,000, i.e. hydroxymethylcellulose, hydroxyethylcellulose, hydroxypropyl methylcellulose.

Appropriate swelling agent may also be selected among the group consisting of polyethylene oxides, polyethylenes, polypropylenes, polyvinyl chlorides, polycarbonates, polystyrenes, polyacrylates and the copolymers thereof. Prefered swelling agents may be selected from carboxyvinyl polymers, polyvinyl alcohols, glucans, scleroglucans, chitosans, mannas, galactomannans, gums, xantan gums, carrageenans, amylase, alginic acid and salts therof, acrylates, methacrylates, acrylic/methacrylic copolymers, polyanhydrides, polyamino acids, methyl vinyl ethers/maleic anhydride copolymers, carboxymethylcellulose and derivatives thereof, ethylcellulose, methylcellulose and derivatives of cellulose in general, superporous hydrogels in general and mixtures thereof.

Most preferred swelling agents are polyethylene oxide with a molecular weight of at least 1,000,000 and hydroxypropyl methylcellulose with a molecular weight of at least 100.000 and combinations thereof.

The amount of swelling agent that is required for the process and forms of the present invention may vary within the range from 1 to 99% by weight based on the total weight of the final composition. The amount of swelling agent preferably starts at 5% by weight and is comprised more preferably within the range of 30-60% by weight. Large amounts of swelling agent allow incorporating large amounts of granulating liquid and of lipophilic agents as well which enhances the final floating properties of the multiparticulate gastro-retentive dosage form.

The intrinsic properties of the solid dosage form according to the invention result from the evaporation of large amounts of liquid comprised within a matrix with a hydrophobic agent upon drying.

As used herein, "lipophilic" in reference to the excipients, is intended to mean any sparingly soluble component that is commonly used in formulation and typically include components with little water solubility or with water insolubility. Without being limitative, an example of a typical little water solubility is less than 1 mg/l.

Suitable lipophilic excipients are not particularly limited, as the invention is surprisingly capable of providing materials showing intrinsic low density and high porosity from the overgranulation of a wide range of different excipients or of mixtures thereof. Preferably, these lipophilic excipients also have hydrophobic properties as they are not capable of binding at all to water molecules. Such excipients are often apolar or show a low polarity, which means that they also do not allow electrostatic interactions with water (such as Keesom forces).

Particular non limiting examples of these excipients are hydrophobic dusty powders, such as silicas, talc, magnesium stearate, as well as general lipidic excipients such as fatty esters, fatty acids, among which stearic acid, or any fatty acid that is solid at room temperature, or mixture thereof. Hydrophobic silica, talc, fatty acid and fatty acid esters or ethers or glicerides are particularly preferred.

Hydrophobic silica possesses physical properties that are useful in a number of applications requiring a high degree of dispersibility, including its use in toner compositions, as antiblocking agents, as adhesion modifiers, and as polymer fillers. Untreated silica particles are hydrophilic due to the presence of silanol groups on the surface of the untreated silica particles. An example of such silica is the commercially available silica Aerosil 200®. Therefore, different degrees of hydrophobicity may be obtained as a result of treatments of the silica, such as with reagents introducing functional apolar groups onto the silica surface, resulting in the reduction the hydrophilic nature of the particles.

Talc, is a mineral composed of hydrated magnesium silicate having the formula $H_2Mg_3(SiO_3)_4$ or $Mg_3Si_4O_{10}(OH)_2$. In loose form it is known as talcum powder and finds uses in cosmetic products, as a lubricant, as a filler in paper manufacture, but also as a food or pharmaceutical additive.

The lipophilic material is generally provided as a powder or "impalpable dust". Powder size $d_{50}$ is generally from 10 nm to 500 $\mu$m, preferably from 10 to 100 nm and most preferably of from 10 to 50 nm. For example, a mixture of talc and hydrophobic silica having a powder size of 15 nm was found to be particularly suitable. The amount of lipophilic excipients will generally be an amount within the range of from about 0.01 to about 90%, preferably within the range of from about 1 to about 60% and more preferably of from about 5 to about 50% by weight of the composition. Indeed proportions of about 5 to about 40%, and even down to about 20% permit high quantities of active ingredient to be loaded in the form and still provide intrinsic properties for the floatability of the final dosage form.

Eventually, further adjuvants may come into the composition of the present form, and may include any of the following components: binder, diluent, lubricant , anti-static agent and optionally other auxiliary agents such as sustained release agents, gelifying agents, disintegrating agents, surfactants. Adjuvants may be of any type, since it is the one or more lipophilic excipients which principally provide through the overgranulation phenomena the resulting floating material. Adjuvants that are particularly useful are those that will create cavities within the structure once dried after the granulation step, and thus, provide a higher porosity to the final form. Examples of such adjuvants are for example, swellable excipients, gelifying agents, protective agent, disintegrant agents, or diluents.

In the framework of this invention, the expression "binder" means any excipients which enhances the linkage between particles and include without being limitative: cellulose derivatives such as methylcellulose, carboxymethylcellulose, carboxypropylcellulose, hydroxypropylcellulose and hydroxypropylmethylcellulose (HPMC), crystalline cellulose, starches or pregelatinized starch, polyvinyl alcohol, polyvinylpyrrolidone (PVP), pullulan, dextrin, acacia, gums, excipients and the like, and combination thereof. PVP is the preferred binder. The amount of binder used in the composition may vary within broad limits, for example from 1 to 20% by weight, preferably from 2 to 10%.

In the framework of this invention, the expression "diluent" means any excipients which acts to dilute the formulation without undergoing a chemical reaction with the formulation components. A diluent of the invention includes generally inert carriers or vehicles, be it crystalline or amorphous. Examples of such diluents are derivatives of sugars, such as lactose, saccharose, mannitol, etc., and mixtures thereof. Hydrolyzed starch (malto-dextrine) can be used, preferably in low amounts.

Certain examples of excipients may be at the same time binder and disintegrants.

Further excipients are disclosed in Handbook of Pharmaceutical excipients, 2nd Ed., 1994, American Pharmaceutical Association, Washington, ISBN 0 91730 66 8, by Wade A., Weller PJ.).

Another aspect of the invention is directed to a novel multiparticulate oral gastro-retentive dosage forms which particles all have an inherent and low density.

The dosage form according to the instant invention may also advantageously contain several active ingredients and combination of active ingredients which may be released according to different dissolution profiles. Indeed, the process according to the instant invention may involve granules which respectively exhibit different rates of dissolution, from immediate to sustained release of the active ingredients (i.e. up to 8 hours). An example of this type of dosage is a combination of ciprofloxacine in sustained release granules together with fast released omeprazole or esomeprazole into the digestive tract.

It is particularly advantageous to use a multiparticulate floating form which avoids the "all or nothing" emptying effect whereas there always remain a majority of particles in the stomach which float into the gastric juice.

The capability to float into the stomach in the gastric juice is usually reached for devices having a density below 1,004. The density provided to the granules is less than about 1, preferably below 0.9 and more preferably below 0.8, depending on the formulation and how far the granulation process is performed and the amount of granulating liquid which is introduced into the process prior to the drying step. Preferably, a dosage according to the invention has a density of about 0.6, and more preferably of about 0.5.

The multiparticulate dosage form resulting from the instant invention may advantageously be adapted to fit a residence time into the stomach based on the active ingredient to be administered, for an utmost therapeutical effect. Thus, it is possible to predetermine the density and thus, the residence time of the gastroretentive forms from the amount of granulating liquid which is involved into the granulation step.

The density can be determined by sinking a solid dosage oral form according to the invention into a liquid having a pH=1.2, or water, or gastric juice or any other liquid of known density. The solid dosage form is sunk at an initial position under a flexible indicator (by a horizontal arrow and a ruler in figure 2) and the deviation it creates allows the calculation of its density according to the following formula:

$$\rho_c = \frac{\rho_f}{1 - \frac{Ewh^3}{4gL^3}\frac{\delta_c}{m_c}}$$

wherein: $\rho_c$ is the tablet density (unit: kg/m$^3$); $\rho_f$ is the liquid density (unit: kg/m$^3$); mc is the tablet weight (unit : kg); $\delta$c is the powder deviation induced by the presence of the tablet (unit : m); L is the truss length (unit : m); w is the truss width (unit : m); h is the truss Thickness (unit : m); E is the coefficient of elasticity of solid (Young's modulus unit : Pa)

The porosity may be calculated from the comparison of the apparent volume with the real volume of the dosage form. While the previous density calculation provides the apparent volume $V_1$ (volume of the matrix with the volume of the pores in the pellet), another measure with a helium pycnometer provides the real volume $V_2$ (without the volume of the pores). Thus, the volume of the pores is given by: $V_p=V_1-V_2$. The porosity is reached by the ratio $V_p/V_1$. Porosity may represent from 10 to 80% of the total volume of the form, and preferably of from 20 to 70% of its volume.

The solid dosage form of the invention may further comprise gas generating and/or bioadhesive agents. Since the particles of the present invention possess inherent low density, they will advantageously float immediately into the stomach after swallowing and thus, do not rely on the use of additional excipients. These may however improve the time residence of the system into the stomach and thus, the bioavailability of the drug to be administered.

The layers may further contain gas forming agent in order to improve the buoyancy. These agents, when in contact with an aqueous media, form a non toxic gas, decrease even more the density of the pharmaceutical form, and provide supplementary floating properties to prolong the gastric residence time into the stomach. Examples of gas forming agents are sodium hydrogen carbonates employed individually or in combination with acids.

Bioadhesive agents may also be incorporated on the outer layer of the solid form, allowing the pharmaceutical form positioning and adhesion to the mucosa of the stomach or the upper gastrointestinal tract.

EXAMPLES

EXAMPLE 1: Preparation of multiparticulate oral gastro-retentive dosage forms according to the invention.

Without being limited to the following example, multiparticulate oral gastro-retentive forms according to the invention can be prepared according to the following process.

On one hand, a powders made of 55 g of paracetamol (the active ingredient) together with 40 g of HPMC and about 5 g of PVP K30 are loaded into a planetary mixer and blended at 150 rpm during 2 min 30 sec. On the other hand, a granulating suspension is prepared by solubilizing about 10g of PVP K30 in a 200 ml water solution comprising 15 g of Aerosil R972. The suspension is prepared by using an Ultra turax mixer.

The granulation is initiated at a rotating speed of 100 rpm by adding the suspension to the powder at a rate of 10 ml/min. Granules are obtained after adding about 130 ml of the solution to the powder. The resulting granules are then dried at a temperature of about 50°C in a ventilated oven until the residual humidity of the granules reaches 10%. At this point, the granules may be sieved on a standard 1 mm mesh size before they are completely dried.

The granules thus obtained may be finally formulated into a sachet, a capsule or compressed into a tablet. For this last form further lubricant aids may be added to the granules to compress the particles into a tablet of 40N hardness.

EXAMPLE 2: Floatability

The floatability of five different types of granules of the following composition (by weight based on the total composition) was tested. Types No 1, 4 and 5 were prepared using a hydrophobic excipient (Aerosil R972) while the types No 2 and 4 were prepared using a hydrophilic excipient (Aerosil 200). The results are provided in the table below.

| Composition (w/w %) | No.1 | No.2 | No.3 | No.4 | No5 |
|---|---|---|---|---|---|
| API: paracetamol | 47.1 | 52.2 | 40 | 40 | 52 |

(continued)

| Composition (w/w %) | No.1 | No.2 | No.3 | No.4 | No5 |
|---|---|---|---|---|---|
| HPMC | 35.4 | | 40 | 40 | |
| MCC | | 31.3 | | | 31 |
| PVP K30 | 8.2 | 9 | 10 | 10 | 9.1 |
| Aerosil R972 | 9.3 | | | 10 | 8 |
| Aerosil 200 | | 7.5 | 10 | | |
| | Floats | Sinks | Sinks | Floats | Floats |

The results thus indicate that the floating properties of the granules cannot be attributed to the sole presence of the swelling agent (i.e. HPMC or MCC) since the dosage forms comprising a hydrophilic material did not float. Thus, the inherent low density of the particles is obtained with the hydrophobic material.

In addition, it appeared that the use of HPMC resulted in forms which had a lower density than those prepared using MCC.

**Claims**

1. A process for making low density particles, comprising the steps of:

   (i) providing a powder mixture comprising a swelling agent;
   (ii) granulating the powder of step (i) with a granulating solution comprising a lipophilic agent into granules;
   (iii) drying the granules of step (ii).

2. The process according to claim 1, further comprising the step (iv) of compressing the granules of step (iii).

3. The process according to anyone of claims 1 to 2, further comprising the step (v) of coating the granules resulting from step (ii) or step (iii).

4. The process according to anyone of claims 1 to 3, wherein the active ingredient is added into the starting powder of step (i) and/or the granulating solution of step (ii), preferably into the starting powder of step (i) and/or is laid on the granules obtained in step (iii).

5. The process according to anyone of claims 1 to 4, wherein a binder is added with the starting material in step (i) and/or the granulating solution of step (ii), preferably into the starting powder of step (i).

6. The process according to anyone of claims 1 to 5, wherein the swelling agent is a cellulose derivative having a molecular weight from 4,000 to 2,000,000, hydroxymethylcellulose, hydroxyethylcellulose hydroxypropyl methylcellulose, superporous hydrogels; polyethylene oxides, polyethylenes; polypropylenes; polyvinyl chlorides; polycarbonates; polystyrenes; polyacrylates; carboxyvinyl polymers; polyvinyl alcohols; glucans; scleroglucans; chitosans; mannas; galactomannans; gums; xantan gums; carrageenans; amylase; alginic acids, acrylates, methacrylates, acrylic/methacrylic copolymers, polyanhydrides, polyamino acids, methyl vinyl ethers/maleic anhydride copolymers, carboxymethylcellulose, carboxymethylcellulose derivatives and copolymers thereof or a water soluble resin and mixture thereof, most preferably selected among polyethylene oxides having a molecular weight of at least 1,000,000 and hydroxypropyl methylcellulose with a molecular weight of at least 100,000 and combinations thereof.

7. The process according to anyone of claims 1 to 6, wherein the granulating solution is an aqueous solution or dispersion, an organic solvent, a hydrophobic liquid or water, preferably water.

8. The process according to anyone of claims 1 to 7, wherein the lipophilic agent comprises one or more highly lipophilic excipients selected from the group consisting of hydrophobic dusty powders and lipidic excipients, preferably from the group consisting of talc, hydrophobic silica, magnesium stearate, glicerides, fatty acid esters or fatty acid, preferably talc, stearic acid glicerides and mixtures thereof.

9. The process according to anyone of claims 1 to 8, wherein the particles comprise:

   - from 0.01 to 90%, preferably 20 to 90% of active ingredient;
   - from 1 to 99%, preferably of swelling agent ;
   - from 1 to 60%, preferably 5 to 50% of lipophilic agent; and optionally
   - from 1 to 20%, preferably 2 to 15% of binder.

10. The process of anyone of claims 1 to 9, wherein the active ingredient is selected from the group consisting of AIDS adjunct agents, alcohol abuse preparations, Alzheimer's disease management agents, amyotrophic lateral sclerosis therapeutic agents, analgesics, anesthetics, antacids, antiarythmics, antibiotics, anticonvulsants, antidepressants, antidiabetic agents, antiemetics, antidotes, antifibrosis therapeutic agents, antifungals, antihistamines, antihypertensives, antiinfective agents, antimicrobials, antineoplastics, antipsychotics, antiparkinsonian agents, antirheumatic agents, appetite stimulants, appetite suppressants, biological response modifiers, biologicals, blood modifiers, bone metabolism regulators, cardioprotective agents, cardiovascular agents, central nervous system stimulants, cholinesterase inhibitors, contraceptives, cystic fibrosis management agents, deodorants, diagnostics, dietary supplements, diuretics, dopamine receptor agonists, endometriosis management agents, enzymes, erectile dysfunction therapeutics, fatty acids, gastrointestinal agents, Gaucher's disease management agents, gout preparations, homeopathic remedy, hormones, hypercalcemia management agents, hypnotics, hypocalcemia management agents, immunomodulators, immunosuppressives, ion exchange resins, levocarnitine deficiency management agents, mast cell stabilizers, migraine preparations, motion sickness products, multiple sclerosis management agents, muscle relaxants, narcotic detoxification agents, narcotics, nucleoside analogs, non-steroidal anti-inflammatory drugs, obesity management agents, osteoporosis preparations, oxytocics,parasympatholytics, parasympathomimetics, phosphate binders, porphyria agents, psychotherapeutic agents, radio-opaque agents, psychotropics, sclerosing agents, sedatives, sickle cell anemia management agents, smoking cessation aids, steroids, stimulants, sympatholytics, sympathomimetics, Tourette's syndrome agents, tremor preparations, urinary tract agents, vaginal preparations, vasodilators, vertigo agents, weight loss agents, Wilson's disease management agents, and mixtures thereof, and preferably is selected from the group consisting of abacavir sulfate, abacavirsulfate / lamivudine / zidovudine, acetazolamide, acetaminophen, acyclovir, albendazole, albuterol, aldactone, allopurinol BP, Aluminium carbonate, Aluminium hydroxide, amoxicillin, amoxicillin / clavulanate potassium, amprenavir, artesunate, atovaquone, atovaquone and proguanil hydrochloride, atracurium besylate, baclofen, barium sulfate, beclomethasone dipropionate, berlactone betamethasone valerat, betaïne, Bismuth subsalicylate, bupropion hydrochloride, bupropion hydrochloride SR, Calcium carbonate, carbamazepin, carbidopa, carvedilol, caspofungin acetate, cefaclor, cefazolin, ceftazidime, céfuroxime, chlorambucil, chloroquin, chlorpromazine, cimetidine, cimetidine hydrochloride, ciprofloxacine, cisatracurium besilate, clobetasol propionate, co-trimoxazole, colfoscerilpalpitate, dextroamphetamie sulfate, dioxin, dihydroxyartemisinin, doxycycline, enalapril maleat, epoprostenol, esomepraxole magnesium, fluticasone propionate, furosemide, gabapentin, glitazones, hydrotalcite, hydrocodone hydrochlorothiazide/triamterene, lamivudine, lamotrigine, levodopa, lithium carbonate, lomefloxacine, losartan potassium, Magnesium aluminate monohydrate melphalan, mercaptopurine, mefloquine mesalazine, metformine, morphin, mupirocin calcium cream, nabumetone, naratriptan, norfloxacine, ofloxacine, omeprazole, ondansetron hydrochloride, ovine, oxiconazole nitrate, oxycodone, paroxetine hydrochloride, pefloxacine, piroxicam, prazodin, prochlorperazine, procyclidine hydrochloride, pyrimethamine, ranitidine bismuth citrate, ranitidine hydrochloride, repaglinide, rofecoxib, ropinirole hydrochloride, rosiglitazone maleat, salmeterol xinafoate, salmeterol, fluticasone propionate, Sodium bicarbonate, sterile ticarcillin disodium/clavulanate potassium, simeticon, simvastatin, spironolactone, statins, succinylcholine chloride, sumatriptan, tapentadol, thioguanine, tirofiban hydrochloride, topotecan hydrochloride, tramadol, tranylcypromine sulfate, trifluoperazine hydrochloride, valacyclovir hydrochloride, vinorelbine, zaleplon, zanamivir, zidovudine, zidovudine or lamivudine, corresponding salts thereof, or mixtures thereof, and is most preferably metformin, glitazones, tramadol, tapentadol, oxycodone, hydromorphone and especially with acetaminophen.

11. The process according to any one of claims 1 to 10, wherein the inherent low density particles have a density below 1, preferably below 0.9 and more preferably below 0.8, and preferably have an intrinsic porosity.

12. The process according to any one of claims 1 to 11, wherein the inherent low density particles are further processed into an oral solid gastro-retentive dosage form in a tablet, a capsule or a sachet.

13. A multiparticulate oral gastro-retentive dosage form in a tablet, a capsule or a sachet, comprising low-density particles obtainable by the process according to any of the preceding claims.

14. The multiparticulate sustained release dosage form according to claim 13 in the form of a tablet.

**15.** The multiparticulate sustained release dosage form according to any of claims 13 or 14 wherein the particles have a porosity of from 10 to 80%, preferably of from 20 to 70% of the volume of the form.

FIGURE 1

EP 2 444 064 A1

Europäisches Patentamt
European Patent Office
Office européen des brevets

EUROPEAN SEARCH REPORT

Application Number

EP 10 29 0570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 133 071 A1 (UNIV AIX MARSEILLE II [FR]) 16 December 2009 (2009-12-16) * the whole document * * claims 1-20; figure 1 * | 1-15 | INV. A61K9/00 |
| X | WO 01/58424 A1 (WEST PHARM SERV DRUG RES LTD [GB]; WATTS PETER JAMES [GB]; SMITH ALAN) 16 August 2001 (2001-08-16) * the whole document * * claims 1-22 * | 1-15 | |
| X | EP 0 235 718 A2 (EISAI CO LTD [JP]) 9 September 1987 (1987-09-09) * the whole document * * claims 1-5 * | 1-15 | |
| X | US 5 626 876 A (MUELLER WALTER [DE] ET AL) 6 May 1997 (1997-05-06) * the whole document * * claims 1-15 * | 1-15 | |
| X | WO 2009/153632 A1 (UNIV WITWATERSRAND JOHANNESBUR [ZA]; PILLAY VINESS [ZA]; CHOONARA YAHY) 23 December 2009 (2009-12-23) * the whole document * * claims 1-45 * | 1-15 | TECHNICAL FIELDS SEARCHED (IPC) A61K |

The present search report has been drawn up for all claims

| Place of search | Date of completion of the search | Examiner |
|---|---|---|
| Munich | 14 April 2011 | Felder, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

15

**ANNEX TO THE EUROPEAN SEARCH REPORT
ON EUROPEAN PATENT APPLICATION NO.**

EP 10 29 0570

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-2011

| Patent document cited in search report | | Publication date | Patent family member(s) | | Publication date |
|---|---|---|---|---|---|
| EP 2133071 | A1 | 16-12-2009 | CA<br>EP<br>WO | 2727206 A1<br>2303241 A1<br>2009150514 A1 | 17-12-2009<br>06-04-2011<br>17-12-2009 |
| WO 0158424 | A1 | 16-08-2001 | AU | 3202001 A | 20-08-2001 |
| EP 0235718 | A2 | 09-09-1987 | AT<br>CA<br>DE<br>ES<br>GR<br>JP<br>US | 68969 T<br>1288697 C<br>3774148 D1<br>2051698 T3<br>3003031 T3<br>62195323 A<br>4844905 A | 15-11-1991<br>10-09-1991<br>05-12-1991<br>01-07-1994<br>17-02-1993<br>28-08-1987<br>04-07-1989 |
| US 5626876 | A | 06-05-1997 | NONE | | |
| WO 2009153632 | A1 | 23-12-2009 | EP | 2306983 A1 | 13-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4814179 A
- WO 8906956 A
- EP 2133071 A

### Non-patent literature cited in the description

- **ALEXANDER STREUBEL ; JUERGEN SIEPMANN ; ROLAND BODMEIER.** Gastroretentive drug delivery systems. *Expert Opin. Drug Deliv.,* 2006, vol. 3 (2), 217-233
- **BARDONNET.** Gastroretentive dosage forms: Overview and special case of Helicobacter Pylori. *J. Control. Rel.,* 2006, vol. 111, 1-18
- **KRÖGEL ; BODMEIER.** Development of a multifunctional matrix drug delivery system surrounded by an impermeable cylinder. *J. Control. Release,* 1999, vol. 61, 43-50
- **STREUBEL ; SIEPMANN ; BODMEIER.** Floating matrix tablets based on low density foam powder. *Eur. J. Pharm. Sci.,* 2003, vol. 18, 37-45
- *Int. J. Pharm.,* 2002, vol. 241, 279-292
- **BULGARELLI et al.** Effect of matrix composition and process conditions on casein-gelatin beads floating properties. *Int. J. Pharm.,* 2000, vol. 198, 157-165
- **TALUKDER ; FASSIHI.** Gastroretentive delivery systems: hollow beads. *Drug Dev. Ind. Pharm.,* 2004, vol. 30, 405-412
- **WADE A. ; WELLER PJ.** Handbook of Pharmaceutical excipients. American Pharmaceutical Association, 1994